# EUROPÄISCHE PATENTSCHRIFT

(11) **EP 2 992 861 B1**
(45) Veröffentlichungstag und Bekanntmachung des Hinweises auf die Patenterteilung: **24.07.2019**
(21) Anmeldenummer: 15184045.1
(22) Anmeldetag: 07.09.2015
(51) Int. Cl.: A61F 5/01

(54) **ORTHESE UND SYSTEM ZUR STÜTZUNG DES FUSSES**
ORTHOSIS AND SYSTEM FOR SUPPORTING THE FOOT
ORTHESE ET SYSTEME DE SOUTIEN DU PIED

(30) Priorität: 08.09.2014 DE 202014104234 U
(43) Veröffentlichungstag der Anmeldung: 09.03.2016
(73) Patentinhaber: Gröhninger, Frank Friedrich, 66450 Oberbexbach (DE)
(72) Erfinder: Gröhninger, Frank Friedrich, 66450 Oberbexbach (DE)
(74) Vertreter: Vièl, Christof

(56) Entgegenhaltungen:
- WO-A1-2005/097014
- WO-A1-2009/139019
- US-A- 3 504 668
- US-A- 3 680 549

## Beschreibung

Die Erfindung bezieht sich auf eine Orthese sowie ein System zur Stützung des Fußes, insbesondere bei Peroneuslähmung in Folge einer Schädigung des Peroneusmuskels oder des Peroneusnervs, wobei die Orthese aus einer relativ starren Vorfußplatte, einem federelastischen Verbindungselement und einem relativ starren Unterschenkelteil besteht.

Derartige Lähmungen entstehen insbesondere bei Schädigungen des Peroneusmuskels bzw. des diesen steuernden Peroneusnervs, wie sie z. B. durch die Folgen eines Schlaganfalls, bei Multiple Sklerose oder Bandscheibenvorfall bedingt sein können.

Die Funktionsstörung (Lähmung oder Teillähmung) wirkt sich dahingehend aus, dass die betroffenen Personen den Fuß nicht mehr bewusst steuern können und dadurch der Fuß, der Schwerkraft folgend, nach unten fällt. Das obere Sprunggelenk kippt in Beugerichtung ab und die Zehenspitzen hängen herab, weshalb beim Gehen in der Schwungphase der gesamte Fuß so weit angehoben werden muss, dass die hängende Fußspitze nicht auf dem Boden schleift, bzw. diesen streift oder an diesem hängen bleibt. Aufgrund dieses Herabhängens des Fußes entsteht eine unphysiologische Beinlängendifferenz, die einen erheblichen Kraftaufwand erfordert und zu einem unharmonischen Gangbild bzw. Sturzgefahr führt.

Diese Funktionsstörung der vorfußhebenden Muskulatur wird als Fußheberschwäche (Fallfuß) bezeichnet. Das aus dieser muskulären Funktionsstörung resultierende Gangbild nennt man Steppergang.

Orthesen zur Stützung des Fußes bei Lähmung des Peroneusmuskels bzw. des diesen steuernden Peroneusnervs sind bekannt.

Es ist bekannt, bei bestimmten Lähmungen den Fuß durch eine Orthese zu fixieren, deren Schienbeinanlage und Fußauflage entweder starr und unbeweglich oder über Verbindungselemente, wie beispielsweise Blattfedern, miteinander verbunden sind.

Bei einer dieser Ausführungsformen ist das Unterschenkelteil unbeweglich mit der Fußauflage verbunden, wobei die Fußauflage und die Schienbeinanlage einen Winkel von ca. 90° einschließen. Hierdurch ist der Fuß starr fixiert. Der Nachteil hierbei ist, dass die Orthese keine M-L- bzw. A-P-Bewegung zulässt. Somit lässt dieser Orthesentyp auch keine Kompensationsbewegungen zur Bodenreaktionskraft zu. Lediglich ein Abrollen der Zehen ist möglich. Durch diese Einschränkung besteht die Gefahr einer Thrombose.

Bei anderen Ausführungsformen ist die Fußauflage, die die gesamte Fußsohle bedeckt über seitliche Federn mit dem Unterschenkelteil verbunden. Bei diesen Orthesen ist eine Anterior-Posterior-Knöchelbewegung zwar prinzipiell möglich, jedoch können diese Orthesen nur in Verbindung mit einem Schuh getragen werden. Barfuß Laufen ist ausgeschlossen.

Die WO 2009/139019 A1 offenbart eine Orthese zur Stützung des Fußes, bestehend aus einer relativ starren Vorfußplatte, wobei die Vorfußplatte zur Aufnahme der Zehengrundgelenke D1-D5 sowie der Zehen ausgebildet ist, einem federelastischen Verbindungselement und einem relativ starren Unterschenkelteil. Da die Vorfußplatte sowohl die Zehengrundgelenke D1-D5 sowie die Zehen stützt, kann die Orthese nur in Verbindung mit einem Schuh getragen werden. Barfuß Laufen ist ausgeschlossen. Die WO 2005/097014 A1 offenbart einen orthopädischen Schuh, bestehend aus einer Sohle, einem Fußstützelement und einem Fußgelenk-Orthesenelement.

Hiervon ausgehend liegt der Erfindung die Aufgabe zu Grunde, eine Orthese sowie ein System zur Stützung des Fußes, insbesondere bei Peroneuslähmung oder Peroneusteillähmung in Folge einer Schädigung des Peroneusmuskels oder des Peroneusnervs (bis hin zum Ausfall der vorfußhebenden Muskulatur), so auszugestalten, dass der Fuß unterstützt wird und die Orthese für verschiedene Schuhe bzw. Gelände einsetzbar ist.

Erfindungsgemäß wird die Aufgabe für eine Orthese zur Stützung des Fußes, insbesondere bei Peroneuslähmung in Folge einer Schädigung des Peroneusmuskels oder des Peroneusnervs, bestehend aus einer relativ starren Vorfußplatte, wobei die Vorfußplatte weniger als 45 % der Fußsohlenfläche bedeckt, einem federelastischen Verbindungselement und einem relativ starren Unterschenkelteil, dadurch gelöst, dass die Vorfußplatte lediglich im Bereich der Zehengrundgelenke angeordnet ist und lediglich zur Aufnahme der Zehengrundgelenke D2-D5 ausgebildet ist.

Weiterhin wird die Aufgabe für eine Orthese zur Stützung des Fußes, insbesondere bei Peroneuslähmung in Folge einer Schädigung des Peroneusmuskels oder des Peroneusnervs, bestehend aus einer relativ starren Vorfußplatte, wobei die Vorfußplatte weniger als 45 % der Fußsohlenfläche bedeckt, einem federelastischen Verbindungselement und einem relativ starren Unterschenkelteil, dadurch gelöst, dass die Vorfußplatte lediglich im Bereich der Zehengrundgelenke angeordnet ist und die Vorfußplatte lediglich zur Aufnahme der Zehengrundgelenke D4-D5 ausgebildet ist.

Für beide Lösungen ist vorteilhaft vorgesehen, dass das Verbindungselement das Unterschenkelteil, das auch ein Schienbeinteil sein kann, mit der Vorfußplatte verbindet. Das Verbindungselement selbst ist federelastisch ausgebildet, vorzugsweise als ein omnizentrisches Gelenk, wodurch sowohl eine medial-lateral-Bewegung als auch eine posterior-anterior-Bewegung des in der Orthese gestützten Gelenks ermöglicht wird. Es ist hierbei ebenfalls vorstellbar, dass nicht nur das Verbindungselement, sondern auch das Schienbeinteil und/oder die Vorfußplatte federelastisch, beispielsweise als omnizentrisches Gelenk, ausgebildet sind. Es ist ebenfalls
vorstellbar, dass das Verbindungselement als eine Einheit zwischen einer relativ starren Vorfußplatte und einem relativ starren Unterschenkelteil bzw. Schienbeinteil angeordnet ist.

Relativ starr bedeutet in diesem Zusammenhang, dass die Vorfußplatte und das Unterschenkelteil bei Druck- und Zugbelastung stabil biegefähig sind, wobei sie jedoch aufgrund der Rückstellkraft im Anschluss an den Abrollvorgang des Fußes in die Ausgangsstellung zurückgehen.

Die Verbindung des Verbindungselementes mit Vorfußplatte ist vorzugsweise im Bereich des Mittelfußes auf der Fußsohlenebene angeordnet. Insbesondere ist vorgesehen, dass die Verbindung des Verbindungselementes mit der Vorfußplatte auf der Medialseite, vorzugsweise im Bereich des Mittelfußes proximal zu dem D1 Grundgelenk auf der Fußsohlenebene, angeordnet ist. Das Verbindungselement kann hierbei von dem Unterschenkelteil zu der Vorfußplatte entlang der Bein/Fuß-Innenseite (medial), der Bein/Fuß-Außenseite (lateral), der Bein/Fuß-Hinterseite (posterior) oder, wenn das Verbindungselement gespalten ist sowohl an der Bein/Fuß-Innenseite als auch an der Bein/Fuß-Außenseite (medial und lateral) oder sowohl an der Bein/Fuß-Vorderseite als auch an der Bein/Fuß-hinterseite (frontal und distal) verlaufen. Dementsprechend, wo das Verbindungselement an der Vorfußplatte angeordnet ist, kann eine Verdrehung des Fußes in die Richtung des Verbindungselementes verhindert werden, da das Verbindungselement den Fuß auch stützt bzw. diesen stabilisiert. Durch das Verbindungselement kann eine federelastische Stützung des Fußes erreicht werden, wobei eine Hebung des Vorfußes durch die Vorfußplatte gegenüber dem Unterschenkelteil bzw. dem Schienbeinteil in verschiedenen Winkelgeraden ermöglicht wird. Beispiele für vorab eingestellte Winkelgrade sind beispielsweise 70°, 80° oder 90°. Da die Orthese an sich oder auch nur das Verbindungselement federelastisch ausgebildet sind, können die vorab eingestellten Winkelgrade durch Belastung eine Bewegungsvarianz aufweisen. Diese Varianz kann bis zu 20° betragen. Das Verbindungselement wirkt ebenfalls, entsprechend der belastungsdynamischen Ausbildung und damit der verschiedenen möglichen Winkelgraden, im Stehen kniebeugehemmend, da das Unterschenkelteil gegenüber der Vorfußplatte nur in den vorgesehenen Winkelgraden bewegbar ist.

Das Unterschenkelteil bzw. Schienbeinteil ist vorzugsweise schalenförmig oder als anatomische Anlage ausgebildet, so dass es nach Anlegen formschlüssig an dem Bein bzw. dem Schienbein anliegt. Hierbei ist ebenfalls vorgesehen, dass das Schienbeinteil in der Frontalebene im Wesentlichen biegesteif ausgeführt sein kann, so dass eine feste Anlage entlang des Längserstreckung des Schienbeins erfolgt. Durch die im Wesentlichen biegesteife Ausgestaltung in der Frontalebene kann eine gute Kraftübertragung von der Vorfußplatte über das Verbindungselement auf das Schienbeinteil erfolgen, ohne dass es zu einer punktuellen Belastung im Schienbeinbereich kommt. Jedoch kann das Unterschenkelteil an seinen Endbereichen auch in medialer und/oder lateraler Richtung elastisch ausgebildet sein, so dass Volumenschwankungen während des Tragens Rechnung getragen werden kann oder auch eine Anpassung an verschiedene Nutzer ermöglicht wird bzw. das Unterschenkelteil zum Befestigen aufgebogen werden kann. Das Unterschenkelteil kann als einstückiges Unterschenkelteil ausgebildet sein, an dem ein Verbindungselement oder ein gespaltenes Verbindungselement angeordnet ist. Es ist jedoch auch vorstellbar, dass das Unterschenkelteil, wenn das Verbindungselement gespalten ist, ebenfalls gespalten ist. In letzterem Fall kann das gespaltene Unterschenkelteil als zwei Unterschenkelteile oder als zwei über einen Steg miteinander verbundene Unterschenkelteile vorliegen. Hierbei kann der Steg steif oder gelenkig ausgebildet sein. Es ist ebenfalls vorgesehen, dass der Steg in Höhe der Knöchelgelenklinie als federelastisches Gelenk ausgebildet sein kann und/oder einfach oder mehrfach als Druckgegenlager (distal angeordnet) oder kniestreckendes Widerlager (frontal angeordnet) am Unterschenkel ausgebildet ist.

Die Vorfußplatte bedeckt weniger als 45 % der Fußsohlenfläche und ist gemäß Anspruch 1 lediglich im Bereich der Zehengrundgelenke D2-D5 angeordnet. Durch diese Ausgestaltung wird eine stärkere Stützung des Fußes ermöglicht, da vier Zehengrundgelenke gestützt werden. Durch diese ausschließliche Anordnung der Vorfußplatte im Bereich der Zehengrundgelenke, werden die Zehen selbst nicht durch die Vorfußplatte gestützt, was vorteilhaft für das Abrollen während des Gehens ist sowie die Zehen dehnt. Gemäß Anspruch 2 ist vorgesehen, dass die Vorfußplatte lediglich zur Aufnahme der Zehengrundgelenke D4-D5, ausgebildet ist. Die Zehengrundgelenke D4-D5 sind an der Außenseite des jeweiligen Fußes angeordnet. Durch Stützung der Zehengrundgelenke D4-D5 kann das Herabfallen des Fußes insgesamt verhindert werden, wodurch eine kontrollierte Knie-Unterschenkel-Fuß-Steuerung, -Führung und -Stabilisierung erreicht wird.

Für die Orthesen gemäß Anspruch 1 oder Anspruch 2 erlaubt die Ausgestaltung der Vorfußplatte, dass nur ein relativ kleiner Teil der Fußsohle durch die Vorfußplatte bedeckt ist. Hierdurch wird ermöglicht, dass die Vorfußplatte mit
nahezu jedem Schuhwerk getragen werden kann. Da ein Großteil der Fußsohle nicht mit der Vorfußplatte bedeckt ist, kann die die Orthese tragende Person auch barfuß gehen.

Es liegt im Rahmen der Erfindung, dass die Vorfußplatte, das Verbindungselement und das Unterschenkelteil einstückig ausgebildet sind.

Dies ist insbesondere vorteilhaft, da durch diese Ausgestaltung ermöglicht wird, dass die Orthese bestehend aus Vorfußplatte, Verbindungselement und Unterschenkelteil in einem Arbeitsschritt gespritzt oder hergestellt wird. Es wird ebenfalls ermöglicht, dass die Orthese bestehend aus Vorfußplatte, Verbindungselement und Unterschenkelteil nach einem individuellen Abdruck, der von der die Orthese tragenden Person erstellt wurde, hergestellt wird. Dies erlaubt demnach sowohl eine Herstellung nach Maß (beispielsweise durch einen Gipsabdruck), als auch eine anpassbare serienmäßige Konfektionsherstellung (verschiedene Größen) im Sinne eines Halbfertig- oder Fertigteils.

Weiterhin ist erfindungsgemäß vorgesehen, dass die Orthese aus thermisch verformbarem Kunststoff, insbesondere faser- und/oder gewebeverstärktem Kunststoff und/oder mechanisch formbaren Werkstoffen, insbesondere aus einem Glas-Carbonfaser-Gemisch oder einem Kohlefasergemisch, besteht.

Das Orthesenmaterial kann einlagig oder mehrlagig hergestellt sein und die Festigkeit kann entsprechend der Verwendung der Orthese, beispielsweise als Sportorthese oder als Orthese für den Alltag, eingestellt werden. Verschiedene Festigkeitsstufen sind beispielsweise kohlenfaserhart, steif, elastisch, thermoelastisch und flexibel. Natürlich ist es zu der Erfindung gehörig, dass verschiedene Bereiche der Orthese verschiedene Festigkeiten aufweisen.

Es ist ebenfalls vorteilhaft vorgesehen, dass die Orthese aus thermisch verformbarem Kunststoff und/oder thermisch verformbarem faser- und/oder gewebeverstärktem Kunststoff besteht und nach Erwärmen oder mechanischer Bearbeitung individuell an die Form des Schienbeins, des Knöchels und des Fußes anpassbar ist.

Durch die Auswahl des Materials und durch das vor dem erstmaligen Anziehen durchgeführte Anpassen der Orthese an den Fuß und das Schienbein kann einer Druckstellenbildung entgegengewirkt werden. Sollte es dennoch zu einer Druckstellenbildung kommen, kann die Orthese, da sie beispielsweise aus thermisch verformbarem Kunststoff besteht, durch einen Orthopädietechniker durch fachgerechtes Erwärmen und erneutes Anpassen derart umgeformt werden, dass die Druckstelle entfernt ist.

Durch mechanische Bearbeitung bzw. Anpassung (Abschleifen, Schneiden, Fräsen...) ist ebenfalls vorteilhaft vorgesehen, dass die Vorfußplatte, das Verbindungselement oder das Unterschenkelteil an die Wünsche des Patienten (Trägers bzw. Nutzers) angepasst werden kann. Beispielsweise können im Kunststoff oder einer Auflage/Polsterung Perforationen zur Belüftung und Fensterungen zur Druckstellenvermeidung eingebracht werden.

Es liegt im Rahmen der Erfindung, dass die Orthese Mittel zum Befestigen der Orthese an dem Unterschenkel und/oder dem Fuß aufweist.

Diese Mittel zum Befestigen der Orthese können beispielsweise Klettbänder sein, die an entsprechende Aufnahmen, welche an der Orthese angeordnet sind, befestigt werden. Die Klettbänder, die beispielsweise zum Fixieren der Orthese an den Unterschenkel dienen, können elastisch, teilelastisch oder unelastisch ausgebildet sein. Die Mittel zum Befestigen der Orthese können aber auch Bänder mit Laschen sein, die in Vorsprünge, die an der Orthese befestigt sind, eingreifen können. Ebenfalls ist vorstellbar, dass die angezogene Orthese durch eine an dem Unterschenkel und den Fuß angelegte angepasste Fixierungsbandage zusätzlich befestigt wird.

Weiterhin ist vorteilhaft vorgesehen, dass die Fläche der Vorfußplatte der Orthese weniger als 25 % der Fußsohlenfläche bedeckt.

Eine Ausgestaltung der Erfindung sieht vor, dass die Vorfußplatte auf der dem Fuß abgewandten Seite eine Rutschhemmung aufweist.

Die Rutschhemmung kann als Profilierung und/oder als gummielastisches adhäsionsförderndes Mittel ausgebildet sein. Hierdurch wird ermöglicht, dass die die Orthese tragende Person noch sicherer barfuß gehen oder laufen kann, da die Rutschhemmung eine Rutschgefahr mindert.

Die Orthese kann auch als wasserfeste Fußheberorthese mit einer Rutschhemmung ausgebildet sein, so dass sie für Nass- und Feuchträume, beispielsweise in der Dusche, der Sauna, am Strand oder im Schwimmbad genutzt werden kann.

Eine weitere Ausgestaltung der Orthese sieht vor, dass die Vorfußplatte auf der dem Fuß zugewandten Seite wenigstens eine Zehenführungspelotte aufweist.

Dies dient sowohl zur Stabilisierung des Fußes gegenüber der Vorfußplatte, als auch zum Strecken, Entlasten, Dehnen oder Korrigieren der Zehen bzw. der Fußstellung.

Es liegt im Rahmen der Erfindung, dass im Auflagebereich des Unterschenkelteils und/oder das Verbindungselement und/oder der Vorfußplatte eine Auflage aus dämpfendem Material angeordnet ist.

Diese Auflage aus dämpfendem Material kann sowohl eine Polsterung als auch eine dünne Oberflächentexturierung sein.

Die Auflage dient zur Verbesserung der haptischen Wahrnehmung der Orthese auf der Haut des Trägers.

Durch die Auflage wird ebenfalls ermöglicht, dass das Bein gegenüber der Orthese gedämpft ist. Vorteilhaft ist hierbei vorgesehen, dass die Auflage aus dämpfendem Material aus einem temperaturregulierenden Material, das Wärmeentwicklung und Schweißbildung verhindert oder reduziert, ausgebildet ist. Es ist hierbei vorgesehen, dass die Auflage aus künstlich oder natürlich hergestellten Materialien besteht. Durch das dämpfende Material kann ebenfalls einer Druckstelle entgegengewirkt werden. Es ist ebenfalls vorstellbar, dass die Auflage aus dämpfendem Material aus wasserfestem Material hergestellt ist.

Vorteilhaft ist weiterhin vorgesehen, dass die Orthese für Schuhwerk mit einer Sprengung (Höhenunterschied von der Ferse bis zum Vorfuß) von bis zu 40 mm verwendbar ist.

Vorzugsweise ist eine Sprengung bis zu 30 mm vorgesehen.

Die Sprengung ist möglich, da die Vorfußplatte lediglich im Bereich der Zehengrundgelenke angeordnet ist und diese teilweise oder vollständig linear unterstützt. Das Abrollen des Fußes führt zu einer belastungsdynamischen Verbiegung der Vorfußplatte, des federelastischen Verbindungselementes und teilweise Verbiegen des Unterschenkelteils.

Die Aufgabe wird ebenfalls durch ein System, bestehend aus einer orthopädischen Fußeinlage und einer der zuvor beschriebenen Orthesen zur Stützung des Fußes dadurch gelöst, dass die orthopädische Fußeinlage in dem Bereich der Zehengrundgelenke eine Vertiefung zur Aufnahme der Vorfußplatte aufweist.

Die Aufgabe wird ebenfalls durch ein System, bestehend aus einer orthopädischen Fußeinlage und einer Orthese zur Stützung des Fußes, wobei die Orthese aus einer Vorfußplatte, einem federelastischen Verbindungselement und einem Unterschenkelteil besteht, dadurch gelöst, dass die orthopädische Fußeinlage in dem Bereich der Zehengrundgelenke eine Vertiefung aufweist, die zur Aufnahme einer Vorfußplatte ausgebildet ist, welche weniger als 45 % der Fußsohlenfläche bedeckt, im Bereich der Zehengrundgelenke angeordnet ist und mindestens zur Aufnahme der Zehengrundgelenke D4-D5 ausgebildet ist.

Weitere Einzelheiten, Merkmale und Vorteile der Erfindung werden für Ausführungsbeispiele, jedoch nicht darauf beschränkt, anhand der Zeichnungen näher erläutert. Es zeigen:
- Fig. 1: eine erste Ausgestaltung einer an einem Fuß getragenen erfindungsgemäßen Orthese in Seitenansicht,
- Fig. 2: eine weitere Ausgestaltung einer an einem Fuß getragenen erfindungsgemäßen Orthese in Seitenansicht,
- Fig. 3: eine Fußsohle mit einer Vorfußplatte, wobei die Vorfußplatte mindestens zur Aufnahme der Zehengrundgelenke D2-D5 geeignet ist,
- Fig. 4: ein System, bestehend aus einer orthopädischen Fußeinlage und einer Orthese zur Stützung des Fußes.

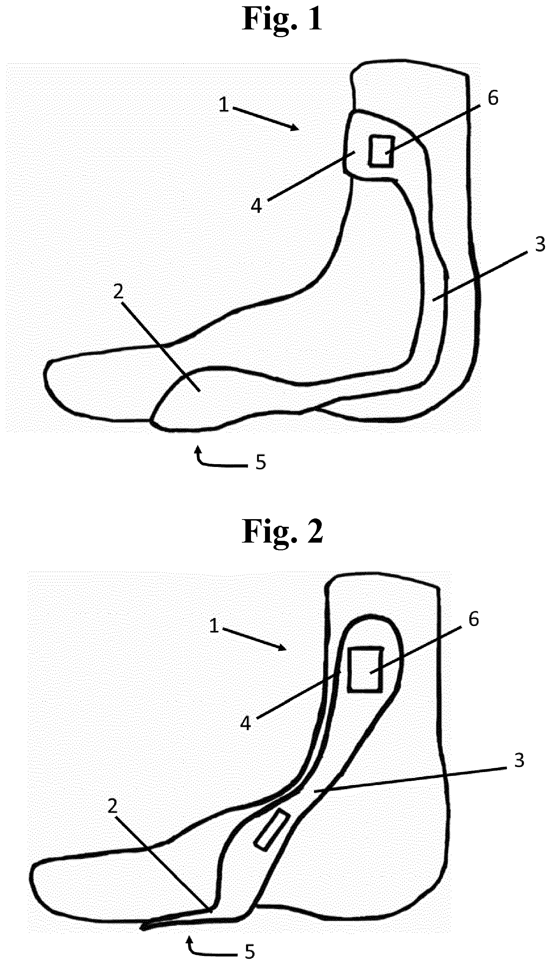

In Fig. 1 ist eine Orthese (1) zur Stützung des Fußes, insbesondere bei Peroneuslähmung in Folge einer Schädigung des Peroneusmuskels oder des Peroneusnervs, bestehend aus einer Vorfußplatte (2), einem Verbindungselement (3) und einem Unterschenkelteil (Schienbeinteil) (4) in Seitenansicht dargestellt. Wie dargestellt, bedeckt die die Vorfußplatte (2) weniger als 45 % (hier ca. 30 %) der Fußsohlenfläche. Die Vorfußplatte (2) ist lediglich im Bereich der Zehengrundgelenke (5) D4-D5 angeordnet. Wie dargestellt, ist das Schienbeinteil (4) schalenförmig bzw. als anatomische Anlage ausgebildet, so dass es nach Anlegen formschlüssig an dem Bein bzw. dem Schienbein anliegt. Das Verbindungselement (3) verbindet das Schienbeinteil (4) mit der Vorfußplatte (2). Die Verbindung des Verbindungselementes (3) mit der Vorfußplatte (2) ist im Bereich des Mittelfußes auf der Fußsohlenebene angeordnet. Das Verbindungselement (3) verläuft von dem Schienbeinteil (4) zu der Vorfußplatte (2) entlang der Bein/Fuß-Innenseite und der Bein/Fuß-Außenseite. Dabei verläuft das Verbindungselement (3) von der Vorfußplatte (2) hinter dem Fußknöchel (zur Ferse hin) entlang, hin zu dem Schienbeinteil (4). Es ist bei der dargestellten Ausführungsform vorgesehen, dass das Verbindungselement (3) gespalten ist und sowohl an der Bein/Fuß-Innenseite als auch an der Bein/Fuß-Außenseite verläuft. Im dargestellten Ausführungsbeispiel soll das Verbindungselement (3) sowohl auf der Bein/Fuß-Außenseite als auch auf der Bein/Fuß-Innenseite an der Vorfußplatte (2) angeordnet sein, wodurch eine Verdrehung des Fußes in die Richtung des Verbindungselementes (3) verhindert wird, da das Verbindungselement (3) den Fuß auch stützt bzw. diesen stabilisiert und diesen führt. Das Verbindungselement (3) ist selbst ein omnizentrisches Gelenk. Es ist ebenfalls vorstellbar (nicht dargestellt), dass ein Bereich des Verbindungselementes (3) aus einem anderen Material hergestellt ist. Dieser Bereich wirkt dann ebenfalls als ein omnizentrisches Gelenk. Es ist ebenfalls vorstellbar (nicht dargestellt), dass ein Bereich des Verbindungselementes (3) ein zusätzliches ominizentrisches oder monozentrisches Gelenk aufweist. Wie dargestellt, weist die Orthese (1) Mittel zum Befestigen der Orthese (1) an dem Unterschenkel und/oder dem Fuß auf. Mittel zum Befestigen der Orthese können beispielsweise Klettbänder sein, die an entsprechende Aufnahmemitteln (6), welche an der Orthese angeordnet sind, befestigt werden. Die Mittel zum Befestigen der Orthese können aber auch Bänder mit Laschen sein, die in Vorsprünge (6), die an der Orthese befestigt sind, eingreifen können. Weiterhin ist dargestellt, dass die Vorfußplatte (2), das Verbindungselement (3) und das Schienbeinteil (4) einstückig ausgebildet sind.

In Fig. 2 ist eine weitere Ausgestaltung der Orthese (1) zur Stützung des Fußes bestehend aus einer Vorfußplatte (2), einem Verbindungselement (3) und einem Unterschenkelteil (4) in Seitenansicht dargestellt. Wie dargestellt, bedeckt die die Vorfußplatte (2) weniger als 45 % der Fußsohlenfläche. Die Vorfußplatte (2) ist lediglich im Bereich der Zehengrundgelenke (5) D2-D5 angeordnet. Wie dargestellt, ist das Unterschenkelteil (4) schalenförmig bzw. als anatomische Anlage ausgebildet, so dass es nach Anlegen formschlüssig an dem Bein anliegt. Das Verbindungselement (3) verbindet das Unterschenkelteil (4) mit der Vorfußplatte (2). Die Verbindung des Verbindungselementes (3) mit der Vorfußplatte (2) ist im Bereich des Mittelfußes auf der Fußsohlenebene angeordnet. Das gespaltene Verbindungselement (3) verläuft von dem Unterschenkelteil (4) zu der Vorfußplatte (2) entlang der Bein/Fuß-Außenseite und der Bein/Fuß-Außenseite. Dabei verläuft das Verbindungselement (3) von der Vorfußplatte (2) vor dem Fußknöchel (zum Fußspann hin) entlang, hin zu dem Unterschenkelteil (4). Es ist bei der dargestellten Ausführungsform vorgesehen, dass das Verbindungselement (3) und das Unterschenkelteil (4) gespalten sind und sowohl an der Bein/Fuß-Innenseite als auch an der Bein/Fuß-Außenseite verlaufen. Wie dargestellt, weist die Orthese (1) Mittel zum Befestigen der Orthese (1) an dem Unterschenkel und/oder dem Fuß auf. Mittel zum Befestigen der Orthese können beispielsweise Klettbänder sein, die an entsprechende Aufnahmemitteln (6), welche an der Orthese angeordnet sind, befestigt werden. Die Mittel zum Befestigen der Orthese können aber auch Bänder mit Laschen sein, die in Vorsprünge (6), die an der Orthese befestigt sind, eingreifen können.

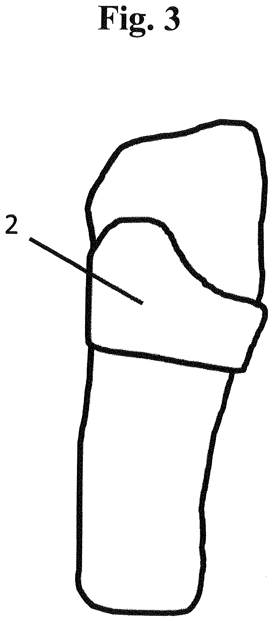

In der Figur 3 ist eine Ausgestaltung der Vorfußplatte (2) in Draufsicht gezeigt. Fig. 3 zeigt hierbei eine Vorfußplatte (2), die weniger als 30 % der Fußsohlenfläche bedeckt und lediglich im Bereich der Zehengrundgelenke angeordnet ist und zur Aufnahme der Zehengrundgelenke D2-D5 ausgebildet ist. Die in Figur 3 dargestellte Ausführungsform erlaubt das Barfußlaufen, wobei der Fuß ohne Strumpf in der Orthese gestützt wird. Es ist jedoch ebenfalls vorgesehen, dass die Orthese mit einem Unterziehstrumpf - mit oder ohne Kompression - getragen werden kann.

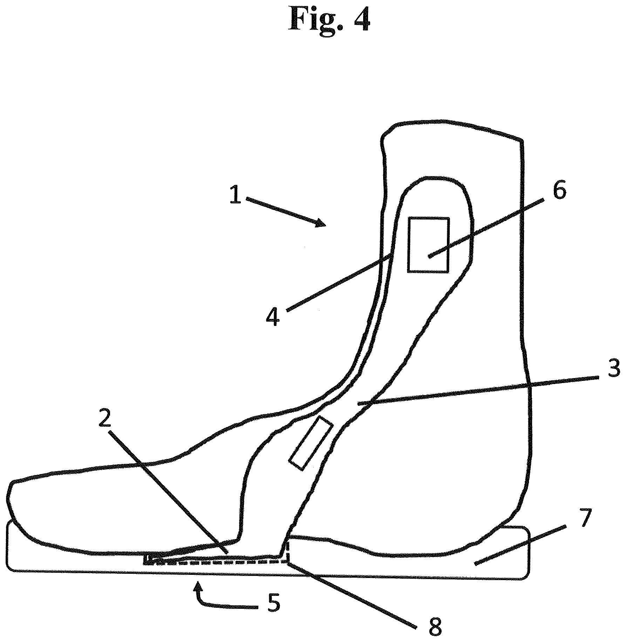

In Fig. 4 ist ein System, bestehend aus einer orthopädischen Fußeinlage (7) und einer Orthese (1) zur Stützung des Fußes, wobei die Orthese (1) aus einer Vorfußplatte (2), einem Verbindungselement (3) und einem Unterschenkelteil (4) besteht, gezeigt. Die Vorfußplatte (2) der Orthese (1) bedeckt weniger als 45 % der Fußsohlenfläche, ist im Bereich der Zehengrundgelenke (5) angeordnet und mindestens zur Aufnahme der Zehengrundgelenke D4-D5 ausgebildet. Die orthopädische Fußeinlage (7) weist in dem Bereich der Zehengrundgelenke (5) eine Vertiefung (8) zur Aufnahme der Vorfußplatte (2) auf. Wie dargestellt, weist die Orthese (1) Mittel zum Befestigen der Orthese (1) an dem Unterschenkel und/oder dem Fuß auf. Mittel zum Befestigen der Orthese können beispielsweise Klettbänder sein, die an entsprechende Aufnahmemitteln (6), welche an der Orthese angeordnet sind, befestigt werden. Die Mittel zum Befestigen der Orthese können aber auch Bänder mit Laschen sein, die in Vorsprünge (6), die an der Orthese befestigt sind, eingreifen können.

## Patentansprüche

1. Orthese (1) zur Stützung des Fußes, insbesondere bei Peroneuslähmung in Folge einer Schädigung des Peroneusmuskels oder des Peroneusnervs, bestehend aus einer relativ starren Vorfußplatte (2), wobei die Vorfußplatte (2) weniger als 45 % der Fußsohlenfläche bedeckt, einem federelastischen Verbindungselement (3) und einem relativ starren Unterschenkelteil (4), **dadurch gekennzeichnet, dass** die Vorfußplatte lediglich im Bereich der Zehengrundgelenke (5) angeordnet ist und lediglich zur Aufnahme der Zehengrundgelenke (5) D2-D5 ausgebildet ist.

2. Orthese (1) zur Stützung des Fußes, insbesondere bei Peroneuslähmung in Folge einer Schädigung des Peroneusmuskels oder des Peroneusnervs, bestehend aus einer relativ starren Vorfußplatte (2), wobei die Vorfußplatte (2) weniger als 45 % der Fußsohlenfläche bedeckt, einem federelastischen Verbindungselement (3) und einem relativ starren Unterschenkelteil (4), **dadurch gekennzeichnet, dass** die Vorfußplatte lediglich im Bereich der Zehengrundgelenke (5) angeordnet ist und die Vorfußplatte (2) lediglich zur Aufnahme der Zehengrundgelenke (5) D4-D5 ausgebildet ist.

3. Orthese (1) zur Stützung des Fußes gemäß Anspruch 1 oder Anspruch 2, **dadurch gekennzeichnet, dass** die Vorfußplatte (2), das Federelement (3) und das Unterschenkelteil (4) einstückig ausgebildet sind.

4. Orthese (1) zur Stützung des Fußes gemäß Anspruch 1, Anspruch 2 oder Anspruch 3, **dadurch gekennzeichnet, dass** die Orthese (1) aus thermisch verformbarem Kunststoff, insbesondere faser- und/oder gewebeverstärktem Kunststoff, und/oder mechanisch formbaren Werkstoffen, insbesondere aus einem Glas-Carbonfaser-Gemisch oder einem Kohlefasergemisch, besteht.

5. Orthese (1) zur Stützung des Fußes gemäß Anspruch 4, **dadurch gekennzeichnet, dass** die Orthese (1) aus thermisch verformbarem Kunststoff und/oder thermisch verformbarem faser- und/oder gewebeverstärktem Kunststoff besteht und nach Erwärmen oder mechanischer Bearbeitung individuell an die Form des Schienbeins, des Knöchels und des Fußes anpassbar ist.

6. Orthese (1) zur Stützung des Fußes gemäß Anspruch 1, Anspruch 2 oder einem der Ansprüche 3 bis 5, **dadurch gekennzeichnet, dass** die Orthese (1) Mittel zum Befestigen der Orthese (1) an dem Unterschenkel und/oder dem Fuß aufweist.

7. Orthese (1) zur Stützung des Fußes gemäß einem der Ansprüche Anspruch 1, Anspruch 2 oder einem der Ansprüche 3 bis 6, **dadurch gekennzeichnet, dass** die Fläche der Vorfußplatte (2) der Orthese (1) weniger als 25 % der Fußsohlenfläche bedeckt.

8. Orthese (1) zur Stützung des Fußes gemäß Anspruch 1 oder Anspruch 2, **dadurch gekennzeichnet, dass** die Vorfußplatte (2) auf der dem Fuß abgewandten Seite eine Rutschhemmung aufweist.

9. Orthese (1) zur Stützung des Fußes gemäß Anspruch 1 oder Anspruch 2, **dadurch gekennzeichnet, dass** die Vorfußplatte (2) auf der dem Fuß zugewandten Seite wenigstens eine Zehenführungspelotte aufweist.

10. Orthese (1) zur Stützung des Fußes gemäß Anspruch 1 oder Anspruch 2, **dadurch gekennzeichnet, dass** im Auflagebereich des Unterschenkelteils (4) und/oder das Verbindungselement (3) und/oder der Vorfußplatte (2) eine Auflage aus dämpfendem Material angeordnet ist.

11. Orthese (1) zur Stützung des Fußes gemäß Anspruch 1 oder Anspruch 2, **dadurch gekennzeichnet, dass** die Orthese (1) für Schuhwerk mit einer Sprengung von bis zu 40 mm verwendbar ist.

12. System, bestehend aus einer orthopädischen Fußeinlage (7) und einer Orthese (1) zur Stützung des Fußes gemäß Anspruch 1 oder Anspruch 2 oder einem der Ansprüche 3 bis 11, **dadurch gekennzeichnet,** die orthopädische Fußeinlage (7) in dem Bereich der Zehengrundgelenke (5) eine Vertiefung (8) zur Aufnahme der Vorfußplatte (2) aufweist.

13. System, bestehend aus einer orthopädischen Fußeinlage (7) und einer Orthese (1) zur Stützung des Fußes, wobei die Orthese (1) aus einer Vorfußplatte (2), einem federelastischen Verbindungselement (3) und einem Unterschenkelteil (4) besteht, **dadurch gekennzeichnet, dass** die orthopädische Fußeinlage (7) in dem Bereich der Zehengrundgelenke (5) eine Vertiefung (8) aufweist, die zur Aufnahme der Vorfußplatte (2) ausgebildet ist, welche weniger als 45 % der Fußsohlenfläche bedeckt, im Bereich der Zehengrundgelenke (5) angeordnet ist und mindestens zur Aufnahme der Zehengrundgelenke (5) D4-D5 ausgebildet ist.

## Claims

1. Orthosis (1) for supporting the foot, in particular in the case of peroneal paralysis as a result of damage to the peroneal muscle or to the peroneal nerve, consisting of a relatively rigid forefoot plate (2), the forefoot plate (2) covering less than 45% of the surface of the sole of the foot, a spring-elastic connecting element (3) and a relatively rigid lower-leg part (4), **characterized in that** the forefoot plate is arranged only in the region of the metatarsophalangeal joints (5) and is designed to receive only metatarsophalangeal joints (5) D2-D5.

2. Orthosis (1) for supporting the foot, in particular in the case of peroneal paralysis as a result of damage to the peroneal muscle or to the peroneal nerve, consisting of a relatively rigid forefoot plate (2), the forefoot plate (2) covering less than 45% of the surface of the sole of the foot, a spring-elastic connecting element (3) and a relatively rigid lower-leg part (4), **characterized in that** the forefoot plate is arranged only in the region of the metatarsophalangeal joints (5) and is designed to receive only metatarsophalangeal joints (5) D4-D5.

3. Orthosis (1) for supporting the foot according to claim 1 or claim 2, **characterized in that** the forefoot plate (2), the spring element (3) and the lower-leg part (4) are in one piece.

4. Orthosis (1) for supporting the foot according to claim 1, claim 2 or claim 3, **characterized in that** the orthosis (1) consists of thermally deformable plastics material, in particular fiber- and/or fabric-reinforced plastics material, and/or mechanically deformable materials, in particular of a glass/carbon fiber mixture or a carbon fiber mixture.

5. Orthosis (1) for supporting the foot according to claim 4, **characterized in that** the orthosis (1) consists of thermally deformable plastics material and/or thermally deformable fiber- and/or fabric-reinforced plastics material and, after heating or mechanical treatment, can be individually adapted to the shape of the shin, the ankle and the foot.

6. Orthosis (1) for supporting the foot according to claim 1, claim 2 or any one of claims 3 to 5, **characterized in that** the orthosis (1) has means for fastening the orthosis (1) to the lower leg and/or the foot.

7. Orthosis (1) for supporting the foot according to any one of claim 1, claim 2 or any one of claims 3 to 6, **characterized in that** the surface of the forefoot plate (2) of the orthosis (1) covers less than 25% of the surface of the sole of the foot.

8. Orthosis (1) for supporting the foot according to claim 1 or claim 2, **characterized in that** the forefoot plate (2) has anti-slip means on the side remote from the foot.

9. Orthosis (1) for supporting the foot according to claim 1 or claim 2, **characterized in that** the forefoot plate (2) has at least one toe-guide pad on the side facing the foot.

10. Orthosis (1) for supporting the foot according to claim 1 or claim 2, **characterized in that** a lining of cushioning material is arranged in the contact region of the lower-leg part (4) and/or the connecting element (3) and/or the forefoot plate (2).

11. Orthosis (1) for supporting the foot according to claim 1 or claim 2, **characterized in that** the orthosis (1) can be used for footwear having a heel-to-toe drop of up to 40 mm.

12. System consisting of an orthopedic foot insert (7) and an orthosis (1) for supporting the foot according to claim 1 or claim 2 or any one of claims 3 to 11, **characterized in that** the orthopedic foot insert (7) has in the region of the metatarsophalangeal joints (5) a recess (8) for receiving the forefoot plate (2).

13. System, consisting of an orthopedic foot insert (7) and an orthosis (1) for supporting the foot, wherein the orthosis (1) consists of a forefoot plate (2), a spring-elastic connecting element (3) and a lower leg part (4), **characterized in that** the orthopedic foot insert (7) has in the region of the metatarsophalangeal joints (5) a recess (8) which is configured to receive the forefoot plate (2), which covers less than 45 % of the surface of the sole of the foot, is arranged in the region of the metatarsophalangeal joints (5) and is designed to receive only the metatarsophalangeal joints (5) D4-D5.

## Revendications

1. Orthèse (1) pour soutenir le pied, en particulier en cas de paralysie fibulaire résultant d'une atteinte du muscle fibulaire ou du nerf fibulaire, constituée d'une plaque d'avant-pied relativement rigide (2), la plaque d'avant-pied (2) couvrant moins de 45 % de la surface de la plante du pied, un élément de liaison à effet ressort (3) et une partie de jambe (4) relativement rigide, **caractérisé en ce que** la plaque d'avant-pied est disposée uniquement dans la région des articulations métatarso-phalangiennes (5) et elle est conçue pour recevoir uniquement les articulations métatarso-phalangiennes (5) D2-D5.

2. Orthèse (1) pour soutenir le pied, en particulier en cas de paralysie fibulaire résultant d'une atteinte du muscle fibulaire ou du nerf fibulaire, constituée d'une plaque d'avant-pied relativement rigide (2), la plaque d'avant-pied (2) couvrant moins de 45 % de la surface de la plante du pied, un élément de liaison à effet ressort (3) et une partie de jambe (4) relativement rigide, **caractérisé en ce que** la plaque d'avant-pied est disposée uniquement dans la région des articulations métatarso-phalangiennes (5) et elle est conçue pour recevoir uniquement les articulations métatarso-phalangiennes (5) D4-D5.

3. Orthèse (1) pour supporter le pied selon la revendication 1 ou la revendication 2, **caractérisée en ce que** la plaque d'avant-pied (2), l'élément à effet ressort (3) et la partie de jambe (4) sont formés d'une seule pièce.

4. Orthèse (1) pour supporter le pied selon la revendication 1, la revendication 2 ou la revendication 3, **caractérisée en ce que** l'orthèse (1) est constituée d'une matière plastique thermoformable, en particulier de matière plastique renforcée de fibres et/ou de tissus, et/ou de matériaux mécaniquement formables, en particulier un mélange de fibres de verre et de carbone ou un mélange de fibres de carbone.

5. Orthèse (1) pour supporter le pied selon la revendication 4, **caractérisée en ce que** l'orthèse (1) est constituée d'une matière plastique thermoformable et/ou d'une matière plastique renforcée de fibres et/ou de tissus et thermoformable et qu'elle peut être adaptée individuellement à la forme du tibia, de la cheville et du pied après chauffage ou traitement mécanique.

6. Orthèse (1) pour supporter le pied selon la revendication 1, la revendication 2 ou l'une des revendications 3 à 5, **caractérisée en ce que** l'orthèse (1) comprend des moyens pour fixer l'orthèse (1) à la jambe inférieure et/ou au pied.

7. Orthèse (1) pour supporter le pied selon la revendication 1, la revendication 2 ou l'une des revendications 3 à 6, **caractérisée en ce que** la surface de la plaque d'avant-pied (2) de l'orthèse (1) couvre moins de 25 % de la surface de la plante du pied.

8. Orthèse (1) pour supporter le pied selon la revendication 1 ou la revendication 2, **caractérisée en ce que** la plaque d'avant-pied (2) présente un effet antidérapant sur le côté opposé au pied.

9. Orthèse (1) pour supporter le pied selon la revendication 1 ou la revendication 2, **caractérisée en ce que** la plaque d'avant-pied (2) présente au moins un coussinet de guidage des orteils du côté dirigé vers le pied.

10. Orthèse (1) pour supporter le pied selon la revendication 1 ou la revendication 2, **caractérisée en ce qu'**un revêtement réalisé en un matériau amortissant est disposé dans la zone d'appui de la partie de jambe (4) et/ou dans l'élément de liaison (3) et/ou dans la plaque d'avant-pied (2).

11. Orthèse (1) pour supporter le pied selon la revendication 1 ou la revendication 2, **caractérisée en ce que** l'orthèse (1) peut être utilisée pour des chaussures ayant une différence de hauteur entre l'avant-pied et le talon pouvant aller jusqu'à 40 mm.

12. Système composé d'une semelle orthopédique (7) et d'une orthèse (1) pour supporter le pied selon les revendications 1 ou 2 ou l'une des revendications 3 à 11, **caractérisé en ce que** la semelle orthopédique (7) présente une dépression (8) dans la zone des articulations métatarso-phalangiennes (5) pour recevoir la plaque d'avant-pied (2).

13. Système composé d'une semelle orthopédique (7) et d'une orthèse (1) pour supporter le pied, l'orthèse (1) étant constituée d'une plaque d'avant-pied (2), d'un élément de liaison à effet ressort (3) et d'une partie de jambe (4) relativement rigide, **caractérisé en ce que** la semelle orthopédique (7) présente une dépression (8) dans la zone des articulations métatarso-phalangiennes (5) pour recevoir la plaque d'avant-pied (2), laquelle couvre moins de 45 % de la surface de la plante du pied, qui est disposée dans la région des articulations métatarso-phalangiennes (5) et qui est conçue pour recevoir au moins les articulations métatarso-phalangiennes (5) D4-D5.
